# FASCICULE DE BREVET EUROPEEN

(11) **EP 0 578 586 B1**
(45) Date de publication et mention de la délivrance du brevet: **09.04.1997**
(21) Numéro de dépôt: 93420279.7
(22) Date de dépôt: 28.06.1993
(51) Int. Cl.: C07C 235/42, C07D 295/18, A01N 37/38

(54) **Dérivés phénylbenzamides fongicides**
Fungizide Phenylbenzamid derivate
Fungicidal Phenylbenzamide derivatives

(30) Priorité: 30.06.1992 FR 9208290
(43) Date de publication de la demande: 12.01.1994
(73) Titulaire: RHONE-POULENC AGROCHIMIE, 69009 Lyon (FR)
(72) Inventeur: Schmitz, Christian, F-69480 Anse (FR); Latorse, Marie Pascale, F-69210 Sourcieux Les Mines (FR)
(74) Mandataire: Chrétien, François

(56) Documents cités:
- EP-A- 0 360 701

## Description

La présente invention a pour objet de nouveaux dérivés phénylbenzamides, leur préparation et leur utilisation dans des compositions fongicides pour la protection des plantes contres les maladies fongiques.

La demande européenne n°0 360 701 décrit un très grand nombre de dérivés amides et notamment des phénylbenzamides ainsi que leur utilisation comme matière active pour la lutte contre les maladies fongiques des plantes. Les exemples montrent en particulier leur activité préventive contre des maladies telles que les mildious.

La demanderesse a maintenant découvert qu'une sélection étroite de ces dérivés présentent les propriétés déjà décrites mais de plus, et de façon surprenante, une telle activité à un excellent niveau ainsi qu' une activité curative remarquable.

Plus précisément l'invention concerne des dérivés phénylbenzamides de formule (I): 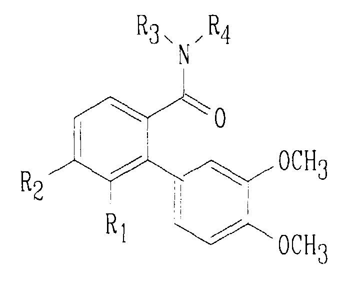 dans laquelle :
- R₁ et R₂, identiques ou différents, sont un atome d'hydrogène ou un radical alkyle, de 1 à 4 atomes de carbone, éventuellement halogéné, ou bien R₁ et R₂ sont chacun un atome de chlore et
- R₃ et R₄, identiques ou différents, sont un alkyle de 1 à 4 atomes de carbone, ou peuvent former, ensemble avec l'atome d'azote qui les porte, un cycle morpholino.

La préparation de ces composés peut s'effectuer selon le procédé décrit dans la demande citée plus haut.

Elle peut être effectuée selon deux autres procédés:

Selon un premier procédé, les composés de formule (I) sont préparés à partir des composés de formule (II) décrite ci-après, après activation de la fonction acide par un agent activateur , de préférence choisi dans le groupe comprenant le chlorure de thionyle, le chlorure de phosphoryle, le trichlorure ou le pentachlorure de phosphore, le dicyclohexylcarbodiimide, le diimidazole carbonyle, les chloroformiates d'alcoyle et l'anhydride trifluoroacétique et puis réaction avec l'amine HNR₃R₄ en présence d'une base organique ou minérale au sein d'un solvant organique de préférence choisi dans le groupe comprenant les solvants chlorés ou aromatiques ou un éther tel que le THF.

Les composés de formule (II) peuvent être préparés par saponification des composés de formule (III) décrite ci-après. La réaction s'effectue au sein d'un alcool tel que l'éthanol en présence d'eau et d'une base minérale telle que de préférence la potasse ou la soude, à une température comprise entre la température ambiante et le reflux du mélange réactionnel. Le mélange réactionnel est alors traité par un acide organique ou minéral tel que de préférence l'acide chlorhydrique pour conduire au composé de formule (II).

La préparation des composés de formule (III), dans laquelle R est de préférence un alkyle de 1 à 4 atomes de carbone, peut s'effectuer par une réaction de couplage arylique entre un composé de formule (IV) décrite ci-après et l'acide 3,4-diméthoxyphénylboronique en présence d'un catalyseur. Pour assurer une bonne régiosélectivité, il est entendu qu'aucun des autres atomes substituants R₁ ou R₂ ne peut être simultanément un atome de brome ou d'iode.

L'acide 3,4-diméthoxyphénylboronique a été préparé par analogie avec les méthodes décrites dans la littérature (Organic Synthesis coll. vol. 4 page 68 ou dans Journal of Organic Chemistry 49 p 5237-5243.

Les esters de formule (IV) sont obtenus par estérification des acides de formule générale (V) décrite ci-après. La réaction s'effectue au sein d'un alcool aliphatique ROH, dans lequel R est un alkyle de 1 à 4 atomes de carbone, tel que le méthanol ou l'éthanol, en présence de quantités variant de 1 à 20 % d'un acide minéral, tel que l'acide chlorhydrique gazeux ou l'acide sulfurique concentré, en général au reflux du mélange réactionnel. Le produit est ensuite isolé par précipitation dans l'eau ou extraction à l'aide d'un solvant organique.

Les acides de formule générale (V) sont obtenus par diazotation des acides anthraniliques correspondants de formule générale (VI) décrite ci-après,selon des méthodes connues.

La préparation des acides anthraniliques de formule (VI) est abondamment décrite dans la littérature.

Dans un deuxième procédé, les composés de formule (I) sont préparés à partir des composés de formule (VII) décrite ci-après, par couplage avec l'acide 3,4-diméthoxy- phénylboronique par une méthode analogue en tout point à celle décrite ci-dessus pour passer des composés de formule (IV) aux composés de formule (III).

Les composés de formule (VII) sont préparés à partir des composés de formule (V) par une méthode analogue en tout point à celle décrite ci-dessus pour passer des composés de formule (II) aux composés de formule (I).

La préparation des composés de formule (V) à partir des acides anthraniliques de formule générale (VI) est décrite ci-dessus dans le premier procédé.

### FORMULES PREMIER PROCEDE

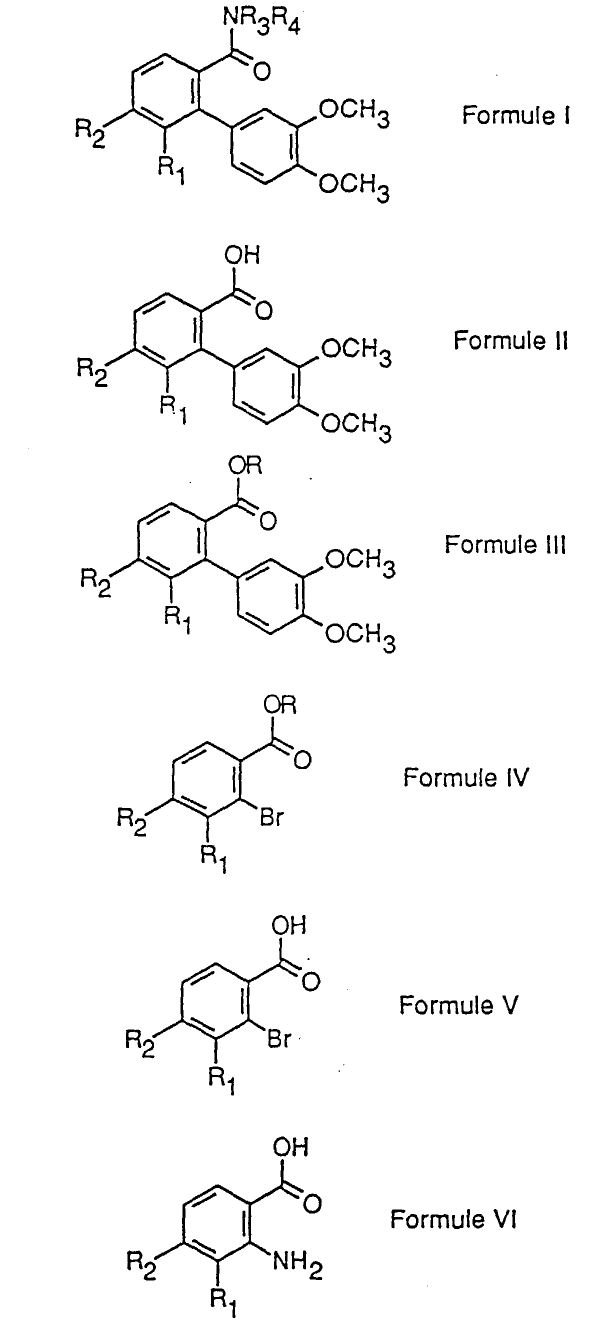

### FORMULES DEUXIEME PROCEDE

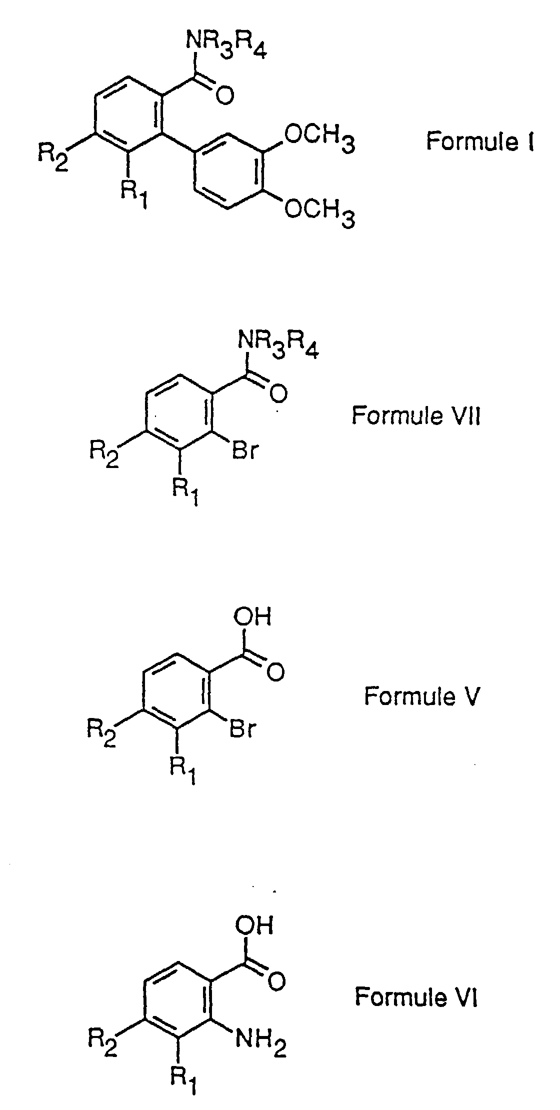

### Exemple 1 : N,N-diéthyl-2-(3,4-diméthoxyphényl)-4-trifluorométhyl benzamide(composé n°1)(Formule I avec R₁= H, R₂=CF₃, R₃=R₄=C₂H₅).

Dans un ballon de 1000 ml, on introduit successivement 500 ml de 1,2-dichloroéthane, 60 g (0,184 mol) d'acide 2-(3,4-diméthoxyphényl)-4-trifluorométhyl benzoïque et 5 ml de N,N-diméthylformamide. Sous agitation et en refroidissant à 0°C, on coule alors goutte à goutte 20 ml de chlorure de thionyle (0,276 mol). Lorsque l'addition est terminée, le mélange réactionnel est chauffé progressivement à 55°C pendant 2 heures puis évaporé à sec. Le résidu est repris par 200 ml de tétrahydrofurane, puis versé goutte à goutte dans une solution contenant 58 ml (0,55 mol) de diéthylamine dans 200 ml de tétrahydrofurane maintenu à une température inférieure à 10°C. Lorsque l'addition est terminée, le mélange réactionnel est agité à température ambiante pendant une heure puis évaporé à sec. Le résidu est repris par du dichlorométhane, lavé successivement par HCl 1N et de l'eau distillée. Après séchage sur sulfate de magnésium, la phase organique est évaporée sous pression réduite pour fournir 62,3 g (rendement: 89 %) de N,N-diéthyl-2-(3,4-diméthoxyphényl)-4-trifluorométhyl benzamide sous forme d'un solide blanc fondant à 109-110°C.

Les deux composés suivants ont été préparés de façon analogue:
* 2-(3,4-diméthoxyphényl)-4-trifluorométhyl-1-morpholinocarbonylbenzène: 60 g (82,5 %) fondant à 130°C (composé n°2) (Formule I avec R₁= H, R₂=CF₃, R₃₊R₄=morpholino).
* N-éthyl-N-méthyl-2-(3,4-diméthoxyphényl)-4-trifluorométhyl benzamide: 71,2 g (81 %) fondant à 103-104°C(composé n°3)(Formule I avec R₁= H, R₂=CF₃, R₃=CH₃, R₄=C₂H₅).

### Exemple 2 : Acide 2-(3,4-diméthoxyphényl)-4-trifluorométhyl benzoïque. (Formule II avec R₁= H, R₂=CF₃).

Dans un ballon de deux litres, on introduit successivement 1000 ml d'éthanol absolu, 290 g (0,82 mol) de 2-(3,4-diméthoxyphényl)-4-trifluorométhyl benzoate d'éthyle et 170 ml (0,164 mol) de lessive de soude 10 N. Le mélange réactionnel est porté au reflux pendant deux heures puis évaporé à sec sous pression réduite. Le résidu est repris par 2,5 litres d'eau et extrait successivement par 500 ml d'acétate d'éthyle et 500 ml de pentane. La phase aqueuse est additionnée de 500 g de glace pilée puis traitée par un excès d'acide chlorhydrique concentré. Le précipité qui se forme est filtré sur verre fritté puis séché sous courant d'air; on obtient ainsi 240,5 g (rendement: 90%) d'acide 2-(3,4-diméthoxyphényl)-4-trifluorométhyl benzoïque sous forme d'un solide beige clair fondant à 194 °C.

### Exemple 3 : 2-(3,4-diméthoxyphényl)-4-trifluorométhyl benzoate d'éthyle(Formule III avec R₁= H, R₂= CF₃, R=C₂H₅).

Dans un ballon de quatre litres, sous atmosphère inerte, on introduit successivement 263 g (0,885 mol) de 2-bromo-4-trifluorométhylbenzoate d'éthyle, 750 ml de 1,2-diméthoxyéthane, 4 g de tétrakistriphénylphosphine palladium, 177 g (0,974 mol) d'acide 3,4-diméthoxyphénylboronique et 1000 ml d'une solution aqueuse de carbonate de sodium 2 M. Le mélange réactionnel est porté au reflux pendant quatorze heures puis évaporé au tiers sous pression réduite. Le mélange réactionnel est versé sur deux litres d'eau; le précipité qui se forme est filtré sur verre fritté, rincé à l'eau puis séché sous courant d'air. On obtient ainsi 294 g (rendement: 94%) de 2-(3,4-diméthoxyphényl)-4-trifluorométhyl benzoate d'éthyle sous forme d'un solide beige fondant à 89°C.

### Exemple 4 : 2-bromo-4-trifluorométhylbenzoate d'éthyle(Formule IV avec R₁ = H, R₂= CF₃, R= C₂H₅).

Dans un ballon de deux litres, on introduit successivement 238 g (0,885 mol) d'acide 2-bromo-4-trifluorométhylbenzoïque 1000 ml d'éthanol absolu et 100 ml d'acide sulfurique concentré. Le mélange réactionnel est porté au reflux pendant 6 heures puis versé, après refroidissement, sur 2,5 litres d'eau glacée; huile formée est extraite à l'acétate d'éthyle. La phase organique est successivement lavée à l'eau, à la soude 1 N puis à nouveau à l'eau. Après séchage sur sulfate de magnésium, le solvant est évaporé pour fournir 263 g (rendement: 100%) de 2-bromo-4-trifluorométhylbenzoate d'éthyle sous forme d'une huile jaune.

### Exemple 5 : Acide 2-bromo-4-trifluorométhylbenzoïque(Formule V avec R₁= H, R₂= CF₃).

Dans un réacteur de trois litres, on introduit successivement 205 g (1 mol) d'acide 4-trifluorométhylanthranilique, 600 ml d'acide acétique glacial et 400 ml d'acide bromhydrique à 47 %. Après dissolution, le mélange réactionnel est refroidi à -10°C dilué par 400 ml d'eau puis traité goutte à goutte par une solution de 69 g (1 mol) de nitrite de sodium dans 200 ml d'eau tout en maintenant la température en dessous de 0°C. Lorsque l'addition est terminée, le mélange réactionnel est agité à 0°C pendant 2 heures. Cette solution est coulée goutte à goutte dans un réacteur de six litres contenant 143,5 g (1 mol) de bromure cuivreux dans 500 ml d'acide bromhydrique à 47 % maintenu à 60°C. Lorsque l'addition est terminée, le mélange réactionnel est agité à 60°C pendant une heure, refroidi à température ambiante puis versé dans deux litres d'eau glacée. Le précipité qui se forme est filtré sur verre fritté, lavé à l'eau puis séché sous courant d'air. On obtient ainsi 216 g (rendement: 80%) d'acide 2-bromo-4-trifluorométhylbenzoïque sous forme d'un solide fondant à 118,5°C.

La préparation de l'acide 4-trifluorométhylanthranilique Formule VI utilisé dans l'exemple 5 est décrite dans la littérature.

### Exemple 6 : deuxième procédé : N-éthyl,N-méthyl-2-(3,4-diméthoxyphényl)-3,4-dichlorobenzamide (composé n°4)(Formule I avec R₁= R₂=Cl, R₃=CH₃, R₄=C₂H₅).

Dans un ballon de 500 ml, sous atmosphère inerte, on introduit successivement 12,4 g (0,040 mol) de N-éthyl,N-méthyl-2-bromo-3,4-dichlorobenzamide, 100 ml de 1,2-diméthoxyéthane, 0,5 g de tétrakistriphénylphosphine palladium, 8 g (0,044 mol) d'acide 3,4-diméthoxyphénylboronique et 60 ml d'une solution aqueuse de carbonate de sodium 2 M. Le mélange réactionnel est porté au reflux pendant quatorze heures puis évaporé au tiers sous pression réduite. Le mélange réactionnel est versé sur deux litres d'eau; le précipité qui se forme est filtré sur verre fritté, rincé à l'eau puis séché sous courant d'air. Après purification par chromatographie, on obtient ainsi 10,3 g (rendement: 70%) de N-éthyl,N-méthyl-2-(3,4-diméthoxyphényl)-3,4-dichlorobenzamide sous forme d'un solide blanc fondant à 102°C.

Le composé suivant a été préparé de façon analogue:
* N,N-diéthyl 2-(3,4-diméthoxyphényl)-3,4-dichlorobenzamide 6,5 g (47,8 %) fondant à 108 °C(composé n° 5)(Formule I avec R₁= R₂=Cl, R₃= R₄=C₂H₅).

### Exemple 7: N-éthyl,N-méthyl-2-bromo-3,4-dichlorobenzamide (Formule VII avec R₁= R₂=Cl, R₃= R₄=C₂H₅).

Dans un ballon de 500 ml, on introduit successivement 200 ml de 1,2-dichloroéthane, 14 g (0,052 mol) d'acide 2- bromo-3,4-dichlorobenzoïque et 2 ml de N,N-diméthylformamide. Sous agitation et en refroidissant à 0°C, on coule alors goutte à goutte 11,5 ml de chlorure de thionyle (0,078 mol). Lorsque l'addition est terminée, le mélange réactionnel est chauffé progressivement à 55°C pendant 2 heures puis évaporé à sec. Le résidu est repris par 50 ml de tétrahydrofurane, puis versé goutte à goutte dans une solution contenant 13 ml (0,15 mol) de N-éthyl,N-méthylamine dans 50 ml de tétrahydrofurane maintenue à une température inférieure à 10°C. Lorsque l'addition est terminée, le mélange réactionnel est agité à température ambiante pendant une heure puis évaporé à sec. Le résidu est repris par du dichlorométhane, lavé successivement par HCl 1N et de l'eau distillée. Après séchage sur sulfate de magnésium, la phase organique est évaporée sous pression réduite pour fournir 12,4 g (rendement: 80 %) de N-éthyl,N-méthyl-2-bromo-3,4-dichlorobenzamide sous forme d'un solide blanc fondant à 71°C.

Le composé suivant a été préparé de façon analogue:
* N,N-diéthyl 2-bromo-3,4-dichlorobenzamide Formule VII avec R₁= R₂= Cl, R₃= R₄= C₂H₅) 13,6 g (rendement: 83,7%).

La préparation de l'acide 2-bromo-3,4-dichlorobenzoïque ( Formule V avec R₁= R₂=Cl) utilisé dans l'exemple 7 a été effectuée comme ci-dessus dans l'exemple 5: on a obtenu le produit sous forme d'une poudre beige 63 g (rendement: 81 %) ,fondant à 196 °C.

### Exemple 8: Test in vivo en curatif en serre sur le midiou de la vigne(Plasmopara viticola):

Des boutures de vigne (Vitis vinifera), de variété Chardonnay sont cultivées dans des godets. Lorsque ces plants sont âgés de 2 mois (stade 8 à 10 feuilles, hauteur 20 à 30 cm), ils sont traités par contamination par pulvérisation au moyen d'une suspension aqueuse de spores de Plasmopara viticola, responsable du mildiou de la vigne, à raison d'environ 5 ml/plant (soit environ 1 x 10⁵ spores par plant).

Après cette contamination, les plants de vigne sont mis en incubation pendant deux jours à 18°C environ en atmosphère saturée d'humidité, puis pendant cinq jours à 20-22°C environ sous 90-100 % d'humidité relative.

Puis les plants contaminés sont traités par pulvérisation au moyen d'une suspension ou solution aqueuse de la matière à tester, à la concentration désirée et contenant un condensat de monooléate de sorbitan et de 20 molécules d'oxyde d'éthylène à concurrence de la moitié de la concentration en matière active. Chaque plant de vigne reçoit environ 5 ml de la solution ou dispersion. Pour chaque concentration de matière active à tester, le traitement est effectué sur deux plants. Des plants contaminés, utilisés comme témoins, sont traités par une solution ne contenant pas de matière active, mais contenant le même condensat de monooléate de sorbitan et d'oxyde d'éthylène à concentration identique.

Après séchage pendant 24 heures, on compare les résultats obtenus dans le cas des plants traités par la matière active à tester à ceux obtenus dans le cas des plants utilisés comme témoin.

Dans ces conditions, on a observé que, à la dose de 110 ppm (0,11 g/l) les composés 1 à 5, entraînaient au moins 95 % d'inhibition du développement du champignon, soit une activité équivalente à celle de la référence commerciale cymoxanyl, prise dans les mêmes conditions.

Ces exemples illustrent bien les propriétés fongicides des composés selon l'invention.

Ceux-ci peuvent en effet être utilisés comme matières actives fongicides, en particulier pour la lutte contre les maladies fongiques des plantes notamment celles dûes aux champignons pathogènes notamment ceux de la famille des oomycètes du type Phytophthora sp par exemple Phytophthora infestans (mildiou de la pomme de terre ou de la tomate), Phytophthora Citrophthora, Phytophthora capsici, Phytophthora cactorum, Phytophthora palmivora, Phytophthora cinnamoni, Phytophthora megasperma, Phytophthora parasitica, Peronospora sp (notamment mildiou du tabac), Plasmopara sp notamment Plasmopara viticola (mildiou de la vigne) et Plasmopara halstedei (mildiou du tournesol), Pseudoperonospora sp (notamment mildiou des cucurbitacées et du houblon), Bremia lactucae (Bremia de la laitue), ainsi que les champignons du sol.

Ils s'appliquent avantageusement à des doses de 0,01 à 5 kg/ha, et plus spécifiquement de 0,02 à 2 kg/ha environ.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active au moins un composé selon l'invention tel que décrit précédemment en association avec les supports solides ou liquides inertes, acceptables en agriculture et/ou les agents tensio-actifs compatibles avec la matière active,également acceptables en agriculture. En particulier sont utilisables les supports inertes et usuels et les agents tensio-actifs usuels.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz.

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthyles. La présence d'au moins un agent tensioactif est généralement indispensable lorsque la matière active et/ou le support inerte ne sont pas solubles dans l'eau et que l'agent vecteur de l'application est l'eau.

Les compositions utilisées dans l'invention peuvent être sous des formes assez diverses, fluides, liquides, ou solides.

Comme formes de compositions fluides, ou liquides, on peut citer notamment les concentrés émulsionnables, les émulsions, les suspensions aqueuses concentrées, les pâtes, les solutions, en particulier les concentrés solubles dans l'eau, les solutions concentrées dans un milieu organique, (solution ULV) et les aérosols.

Les concentrés émulsionnables ou solubles comprennent le plus souvent 10 à 80 % de matière active, les émulsions ou solutions prêtes à l'application contenant, quant à elles, 0,001 à 20 % de matière active. En plus de la matière active et du solvant, les concentrés émulsionnables peuvent contenir, quand c'est nécessaire, un co-solvant approprié et de 2 à 20 % d'additifs appropriés, comme des stabilisants, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

A titre d'exemples, voici la composition de quelques concentrés émulsionnables :

### Exemple CE 1:

- matière active (composé n° 1) 250g/l
- huile végétale epoxydée 25 g/l
- mélange de sulfonate d'alcoylaryle et d'éther de polyglycol et d'alcools gras. 100g/l
- diméthylformamide 50 g/l
- xylène 575 g/l

### Exemple CE 2:

- matière active (composé n° 2) 400 g/l
- dodécylbenzene sulfonate alcalin . 24 g/l
- nonylphénol oxyéthyle à 10 molécules d'oxyde éthylène 16 g/l
- cyclohexanone 200 g/l
- solvant aromatique. qsq 1 l

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble: certaines matières solides organiques ou des sels minéraux peuvent être dissous dans le support pour aider à empêcher la sédimentation ou comme antigels pour l'eau.

A titre d'exemple, voici la composition de plusieurs suspensions aqueuses concentrées selon l'invention :

### Exemple SAC 1 :

On prépare une suspension aqueuse comprenant :
- matière active (composé n° 3) 100 g/l
- agent mouillant (alkylphénol polyéthoxylèné) 5 g/l
- agent dispersant (Naphtalène sulfonate de Na) 10 g/l
- antigel (Propylèneglycol) 100 g/l
- épaississant (Polysaccharide) 3 g/l
- biocide (Formaldéhyde) 1 g/l
- eau Q.S.P.1 litre

### Exemple SAC 2 :

On prépare une suspension comprenant :
- matière active (composé n° 4) 250 g/l
- agent mouillant (alcool synthétique en C13 polyéthoxylé): 10 g/l
- agent dispersant (lignosulfonate de sodium) 15 g/l
- antigel (urée) 50 g/l
- épaississante (Polysaccharide) 2,5 g/l
- biocide (Formaldéhyde) 1 g/l
- eau Q.S.P.1 litre

### Exemple SAC 3 :

On prépare une suspension aqueuse comprenant :
- matière active (composé n°5) 500 g/l
- agent mouillant (alcool synthétique en C13 polyéthoxylé): 10 g/l
- agent dispersant (phosphate de polyarylphénol éthoxylé salifié) 50 g/l
- antigel (propylèneglycol) 100 g/l
- épaissement (polysaccharide) 1,6 g/l
- biocide (méthylhydroxy-4-benzoate de sodium) 3,3 g/l
- eau Q.S.P.1 litre.

Comme formes de compositions solides, on peut citer les poudres pour poudrage (à teneur en matières actives pouvant allerjusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 0,5 et 80 % pour ces derniers cas).

Les poudres mouillables (ou poudres à pulvériser) sont habituellement préparées de manière qu'elles contiennent 10 à 95 % de matière active, et elles contiennent habituellement, en plus du support solide, de 0 à 5 % d'un agent mouillant, de 3 à 10 % d'un agent dispersant, et, quand c'est nécessaire, de 0 à 1O % d'un ou plusieurs stabilisants et/ou autres additifs, comme des agents de pénétration, des adhésifs, ou des agents antimottants, colorants, etc...

A titre d'exemple, voici la composition pondérale de plusieurs poudres mouillables.

### Exemple PM1 :

- matière active (composé n° 1) 10 %
- alcool synthétique oxo de type ramifié, en C13 éthoxyle par 8 à 10 oxyde d'éthylène (agent mouillant) 0,75 %
- lignosulfonate de calcium neutre (agent dispersant 12 %
- carbonate de calcium (charge inerte) qsq 100 %.

### Exemple PM 2 :

- matière active (composé n° 2) 50 %
- alcool gras éthoxylé (agent mouillant) 2,5 %
- styrylphenol ethoxyle (agent dispersant) 5 %
- craie (support inerte) 42,5 %

### Exemple PM 3 :

on utilise les mêmes ingrédients que dans l'exemple précédent, dans les proportions ci-après:
- matière active (composé n° 2) 75 %
- agent mouillant 1,5 %
- agent dispersant 8 %
- carbonate de calcium (charge inerte) qsp 100 %

### Exemple PM4 :

- matière active (composé n° 3) 90 %
- alcool gras éthoxylé (agent mouillant) 4 %
- styrylphenol éthoxylé (agent dispersant) 6 %

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser, dont la mouillabilité et la mise en suspension sont avantageuses; on peut les mettre en suspension avec de l'eau à toute concentration désirée et cette suspension est utilisable très avantageusement en particulier pour l'application sur les feuilles des végétaux.

Les composés de formule (I) peuvent encore être utilisés sous forme de poudres pour poudrage ; on peut aussi utiliser une composition comprenant 50 g de matière active et 950 g de talc; on peut aussi utiliser une composition comprenant 20 g de matière active, 10 g de silice finement divisée et 970 g de talc ; on mélange et broie ces constituants et on applique le mélange par poudrage.

Les granulés pour poudrage ont des dimensions comprises entre O,1 et 2 mm et peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Voici deux exemples de composition de granulé :

| **Exemples G 1 et G 2 :** | | |
|---|---|---|
| - matière active (composé n° 4 | 50 g | 200 g |
| - propylène glycol | 50 g | 50 g |
| - éther de cétyle et de polyglycol | 2,5 g | 2,5 g |
| - polyéthylène glycol | 35 g | 35 g |
| - koalin (granulométrie : 0,3 à 0,8 mm) | 910 g | 760 g. |

Les composés selon l'invention peuvent être avantageusement formulés sous la forme de granulés dispersibles dans l'eau également compris dans le cadre de l'invention.

Ces granulés dispersibles, de densité apparente généralement comprise entre environ 0,3 et 0,6 ont une dimension de particules généralement comprise entre environ 150 et 2000, et de préférence entre 300 et 1500, microns.

La teneur en matière active de ces granulés est généralement comprise entre environ 1 % et 90 % et de préférence entre 25 % et 90 %.

Le reste du granulé est essentiellement composé d'une charge solide et éventuellement d'adjuvants tensio-actifs conférant au granulé des propriétés de dispersibilité dans l'eau. Ces granulés peuvent être essentiellement de deux types distincts selon que la charge est hydrosoluble, elle peut être minérale et de préférence organique. On a obtenu d'excellents résultats avec l'urée. Dans le cas d'une charge insoluble, celle-ci est de préférence minérale, comme par exemple le kaolin ou la bentonite. Elle est alors accompagnée d'agents tensio-actifs (à raison de 2 à 20 % en poids du granule), adjuvants tensio-actifs dont plus de la moitié est constituée par au moins un agent dispersant, essentiellement anionique, tel qu'un poly(naphtalène sufonate alcalin ou alcalino terreux) ou un lignosulfonate alcalin ou alcalino-terreux, le reste étant constitué par des mouillants non ioniques ou anioniques tel qu'un alcoylnaphtalène sulfonate alcalin ou alcalino-terreux.

Par ailleurs, bien que cela ne soit pas indispensable, on peut ajouter d'autres adjuvants tels que des agents anti-mousse.

Le granulé selon l'invention peut être préparé par mélange des ingrédients nécessaires puis granulation selon plusieurs techniques en soi connues (drageoir, lit fluide, atomiseur, extrusion, etc...). On termine généralement par un concassage suivi d'un tamisage à la dimension de particule choisie dans les limites mentionnées ci-dessus.

De préférence, il, est obtenu par extrusion. En opérant comme indiqué dans les exemples ci-après, on a préparé les compositions suivantes de granules dispersibles.

### Exemple GD1 :

Dans un mélangeur, on mélange 90 % en poids de matière active (composé n° 5) et 10 % d'urée en perles. Le mélange est ensuite broyé dans un broyeur à broches. On obtient une poudre humide qui est extrudée dans une extrudeuse à rouleau perforé. On obtient un granule qui est séché, puis concassé et tamisé, de façon à ne garder respectivement que les granules d'une dimension comprise entre 150 et 2000 microns.

### Exemple GD2 :

Dans un mélangeur, on mélange les constituants suivants :
- matière active (composé n° 2) 75 %
- agent mouillant (alkylnaphtalène sulfonate de sodium) 2 %
- agent dispersant (polynaphtalène sulfonate de sodium) 8 %
- charge inerte insoluble dans l'eau (koalin) 15 %

### Exemple GD3 :

- matière active (composé n° 1) 20 %
- alkylnaphtalène sulfonate de sodium 2 %
- méthylène bis naphtalène sulfonate de sodium 8 %
- kaolin 70 %

Ce mélange est granulé en lit fluide, en présence d'eau, puis séché, concassé et tamisé de manière à obtenir des granules de dimension comprise entre 0,16 et O,40 mm.

Ces granulés peuvent être utilisés seuls, en solution ou dispersion dans de l'eau de manière à obtenir la dose cherchée. Ils peuvent aussi être utilisés pour préparer des associations avec d'autres matières actives, notamment fongicides, ces dernières étant sous la forme de poudres mouillables, ou de granulés ou suspensions aqueuses.

Les composés selon l'invention peuvent être encore formulés sous forme de solutions organiques encapsulables, notamment par polymérisation interfaciale, dans des capsules à paroi polymériques, par exemple à base de polyamides de polyurées ou de polyamide urées. Ces capsules se trouvent à l'état de dispersion aqueuse concentrée que l'on peut diluer au moment de l'emploi pour obtenir une bouillie de pulvérisation.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et ils peuvent avoir une consistance épaisse comme celle d'une "mayonnaise".

L'invention concerne de plus un procédé de traitement, tant en curatif qu'en préventif, des végétaux contre les maladies causées par les champignons phytopathogènes notamment ceux de la famille des oomycètes du type Phytophthora sp par exemple Phytophthora infestans (mildiou de la pomme de terre ou de la tomate), Phytophthora Citrophthora, Phytophthora capsici, Phytophthora cactorum, Phytophthora palmivora, Phytophthora cinnamoni, Phytophthora megasperma, Phytophthora parasitica, Peronospora sp (notamment mildiou du tabac), Plasmopara sp notamment Plasmopara viticola (mildiou de la vigne) et Plasmopara halstedei (mildiou du tournesol), Pseudoperonospora sp (notamment mildiou des cucurbitacees et du houblon), Bremia lactucae (Bremia de la laitue), ainsi que les champignons du sol, ce procédé étant caractérisé en ce qu'on applique un dérivé selon l'invention. L'excellente activité curative des composés selon l'invention est particulièrement avantageuse puisqu'elle permet de diminuer le nombre des traitements préventifs systématiques tout en assurant un bon contrôle des parasites.

Ces dérivés peuvent être utilisés comme seule matière active ou en association avec une autre matière active agrochimique, notamment fongicide comme par exemple ceux de la famille des thiocarbamates, des éthylène bis(dithiocarbamates), tels que le thiram, le manèbe, le zinèbe et le mancozèbe, des phthalimides, tels que le captane, le captafol et le folpet, des acylalanines,telles que le metalaxyl, l'oxadixyl et le benalaxyl, des composés à base de cuivre, des dérivés de l'acide phosphoreux comme le phoséthyl-Al, le dithianon, le chlorothalonil, le cymoxanyl, des thiadiazoles, des N,N'-dialcoyl-N-phénylsulfamides.

Ce procédé est caractérisé en ce qu'il consiste à appliquer sur ces végétaux une quantité efficace d'une composition contenant comme matière active un composé selon la formule (I). Par "quantité efficace" on entend une quantité suffisante pour permettre le contrôle et la destruction des champignons présents sur ces végétaux. les doses d'utilisation peuvent toutefois varier dans de larges limites selon le champignon à combattre, le type de culture, les conditions climatiques, et selon le composé utilisé.

En pratique des doses allant de 1 g/hl à 500 g/hl correspondant sensiblement à des doses de matière active par hectare de 10 g/ha à 5000 g/ha environ donnent généralement de bons résultats.

Comme exemples de procédés de traitement utilisables, on peut citer la pulvérisation foliaire ou au sol, le poudrage, le trempage, l'incorporation au sol de granulés, de poudres ou de bouillies, l'arrosage, l'injection dans les arbres, le badigeonnage et le traitement des semences.

## Revendications

1. Composé de formule (I): 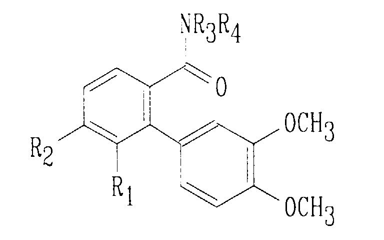 dans laquelle:
- R₁ et R₂, identiques ou différents, sont un atome d'hydrogène ou un radical alkyle, de 1 à 4 atomes de carbone, éventuellement halogéné, ou bien R₁ et R₂ sont chacun un atome de chlore et
- R₃ et R₄, identiques ou différents, sont un alkyle de 1 à 4 atomes de carbone ou peuvent former, ensemble avec l'atome d'azote qui les porte, un cycle morpholino.

2. Composé selon la revendication 1, caractérisé en ce que, dans la formule I, l'un des R₁, R₂ est un atome d'hydrogène et l'autre est le groupe trifluorométhyle.

3. Composé selon la revendication 1, caractérisé en ce que, dans la formule I, R₁, R₂ sont chacun un atome de chlore.

4. Composé selon la revendication 1, caractérisé en ce que, dans la formule I, R₃ et R₄, identiques ou différents, sont un méthyle ou un éthyle.

5. Composé selon la revendication 1, caractérisé en ce que, dans la formule I, R₃ et R₄ forment, ensemble avec l'atome d'azote qui les porte, un cycle morpholino.

6. Composition fongicide pour la protection des plantes, caractérisée en ce qu'elle contient, comme matière active, un composé selon l'une des revendications 1 à 5.

7. Procédé pour la protection des plantes contre les maladies fongiques, caractérisé en ce qu'on applique un composé selon l'une des revendications 1 à 5.

8. Procédé selon la revendication 7), caractérisé en ce qu'on effectue un traitement curatif.

9. Procédé de préparation des composés de formule (I), selon l'une des revendications 1 à 5, caractérisé en ce qu'on traite des composés de formule (II): 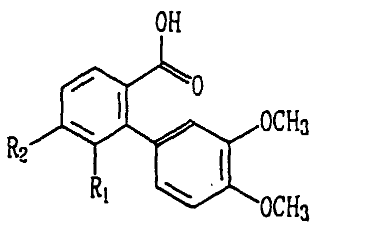 dans laquelle R₁ et R₂ ont la même signification que dans la formule I,
par un agent activateur de leur fonction acide et en ce qu'on fait réagir le produit obtenu avec une amine HNR₃R₄, en présence d'une base organique ou minérale au sein d'un solvant organique.

10. Procédé selon la revendication 9), caractérisé en ce que l'agent activateur de la fonction acide est choisi dans le groupe comprenant le chlorure de thionyle (SOCl₂), le chlorure de phosphoryle (POCl₃), le trichlorure ou le pentachlorure de phosphore (PCl_{3,} PCl₅),
le dicyclohexylcarbodiimide, le diimidazole carbonyle, les chloroformiates d'alcoyle et l'anhydride trifluoroacétique, et en ce que la réaction avec l'amine HNR₃R₄ est effectuée dans un solvant choisi dans le groupe comprenant les solvants chlorés ou aromatiques et des éthers tels que le THF.

11. Procédé selon la revendication 9, caractérisé en ce que les composés de formule (II) sont préparés par saponification des composés de formule (III): 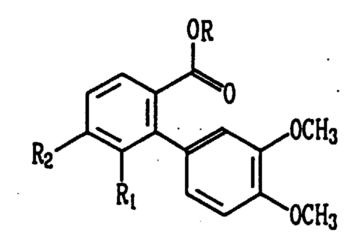 dans laquelle R₁ et R₂ ont la même signification que dans la formule I et R est un alkyle de 1 à 4 atomes de carbone,
au sein d'un alcool tel que l'éthanol en présence d'eau et d'une base minérale alcaline, à une température comprise entre la température ambiante et le reflux du mélange réactionnel, le mélange réactionnel étant ensuite traité par un acide organique ou minéral tel que l'acide chlorhydrique.

12. Procédé selon la revendication 11, caractérisé en ce que les composés de formule (III) sont préparés par une réaction de couplage arylique entre un composé de formule (IV): 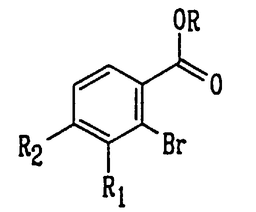 dans laquelle R, R₁ et R₂ ont la même signification que dans la formule III et aucun des substituants R₁, R₂ ne peut être simultanément un atome de brome ou d'iode,
et l'acide 3,4-diméthoxyphénylboronique en présence d'un catalyseur.

13. Procédé pour la préparation des composés de formule (I) caractérisé en ce qu'on effectue une réaction de couplage arylique entre un composé de formule (VII): 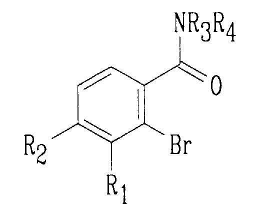 dans laquelle R₁ et R₂ ont la même signification que dans la formule III et ne peuvent être simultanément un atome de brome ou d'iode,
et l'acide 3,4-diméthoxyphénylboronique, en présence d'un catalyseur.

14. Procédé selon la revendication 13, caractérisé en ce que les composés de formule VII sont préparés en traitant des composés de formule (V): 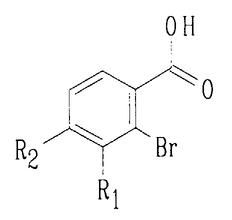 dans laquelle R₁ et R₂ ont la même signification que dans la formule I,
par un agent activateur de leur fonction acide et en ce qu'on fait réagir le produit obtenu avec une amine HNR₃R₄, en présence d'une base organique ou minérale au sein d'un solvant organique.

## Patentansprüche

1. Verbindungen der Formel (I) : 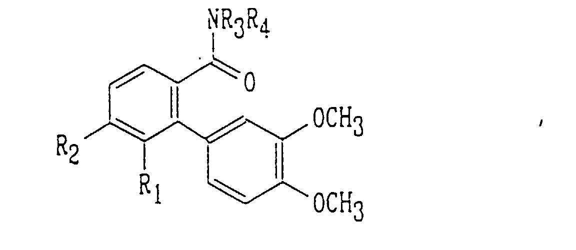 worin bedeuten:
- R₁ und R₂, die gleich oder voneinander verschieden sind, ein Wasserstoffatom oder eine ggf. halogenierte Alkylgruppe mit 1 bis 4 Kohlenstoffatomen, oder R₁ und R₂ jeweils ein Chloratom und
- R₃ und R₄, die gleich oder voneinander verschieden sind, eine Alkylgruppe mit 1 bis 4 Kohlenstoffatomen, oder zusammen mit dem Stickstoffatom, an das sie gebunden sind, einen Morpholinring.

2. Verbindungen nach Anspruch 1, dadurch gekennzeichnet, daß in Formel (I) einer der Substituenten R₁ und R₂ ein Wasserstoffatom und der andere eine Trifluormethylgruppe bedeuten.

3. Verbindungen nach Anspruch 1, dadurch gekennzeichnet, daß in Formel (I) R₁ und R₂ jeweils ein Chloratom sind.

4. Verbindungen nach Anspruch 1, dadurch gekennzeichnet, daß in Formel (I) R₃ und R₄, die gleich oder voneinander verschieden sind, Methyl oder Ethyl bedeuten.

5. Verbindungen nach Anspruch 1, dadurch gekennzeichnet, daß in Formel (I) R₃ und R₄ zusammen mit dem Stickstoffatom, an das sie gebunden sind, einen Morpholinring bilden.

6. Fungizide Zusammensetzung zum Pflanzenschutz, dadurch gekennzeichnet, daß sie als Wirkstoff eine Verbindung nach einem der Ansprüche 1 bis 5 enthält.

7. Verfahren zum Schutz von Pflanzen gegen Pilzerkrankungen, gekennzeichnet durch Anwendung einer Verbindung nach einem der Ansprüche 1 bis 5.

8. Verfahren nach Anspruch 7, dadurch gekennzeichnet, daß eine kurative Behandlung durchgeführt wird.

9. Verfahren zur Herstellung der Verbindungen der Formel (I) nach einem der Ansprüche 1 bis 5, dadurch gekennzeichnet, daß Verbindungen der Formel (II) : 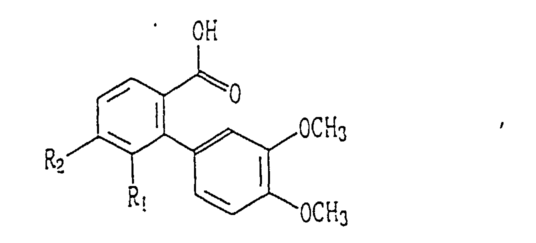 worin R₁ und R₂ die gleiche Bedeutung wie in Formel (I) besitzen, mit einem Mittel zur Aktivierung ihrer Säuregruppe behandelt werden und das erhaltene Produkt in Gegenwart einer organischen oder anorganischen Base in einem organischen Lösungsmittel mit einem Amin HNR₃R₄ umgesetzt wird.

10. Verfahren nach Anspruch 9, dadurch gekennzeichnet, daß das Mittel zur Aktivierung der Säuregruppe unter Thionylchlorid (SOCl₂), Phosphorylchlorid (POCl₃), Phosphortrichlorid oder Phosphorpentachlorid (PCl₃, PCl₅), Dicyclohexylcarbodiimid, Carbonyldiimidazol, Alkylchlorformiaten und Trifluoressigsäureanhydrid ausgewählt wird und die Reaktion mit dem Amin HNR₃R₄ in einem Lösungsmittel durchgeführt wird, das unter chlorierten oder aromatischen Lösungsmitteln und Ethern wie THF ausgewählt ist.

11. Verfahren nach Anspruch 9, dadurch gekennzeichnet, daß die Verbindungen der Formel (II) durch Verseifung der Verbindungen der Formel (III): 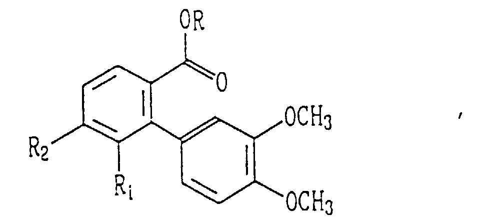 worin R₁ und R₂ die gleiche Bedeutung wie in Formel (I) besitzen und R eine Alkylgruppe mit 1 bis 4 Kohlenstoffatomen bedeutet, in einem Alkohol wie Ethanol in Gegenwart von Wasser und einer anorganischen Alkalibase bei einer Temperatur im Bereich zwischen Raumtemperatur und der Rückflußtemperatur des Reaktionsgemisches hergestellt werden, wobei das Reaktionsgemisch anschließend mit einer organischen oder anorganischen Säure wie Salzsäure behandelt wird.

12. Verfahren nach Anspruch 11, dadurch gekennzeichnet, daß die Verbindungen der Formel (III) durch eine Aryl-Kupplungsreaktion zwischen einer Verbindung der Formel (IV) : 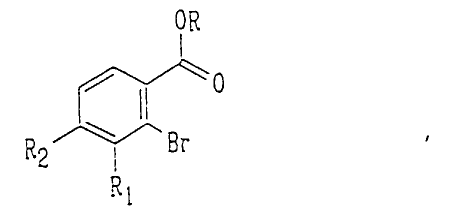 worin R, R₁ und R₂ die gleiche Bedeutung wie in Formel (III) besitzen und die Substituenten R₁ und R₂ nicht gleichzeitig ein Brom- oder Iodatom bedeuten, mit 3,4-Dimethoxyphenylboronsäure in Gegenwart eines Katalysators hergestellt werden.

13. Verfahren zur Herstellung der Verbindungen der Formel (I), dadurch gekennzeichnet, daß eine Aryl-Kupplungsreaktion zwischen einer Verbindung der Formel (VII) : 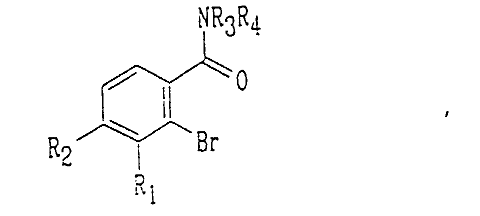 worin R₁ und R₂ die gleiche Bedeutung wie in Formel (III) besitzen und nicht gleichzeitig ein Brom- oder Iodatom bedeuten, und 3,4-Dimethoxyphenylboronsäure in Gegenwart eines Katalysators durchgeführt wird.

14. Verfahren nach Anspruch 13, dadurch gekennzeichnet, daß die Verbindungen der Formel (VII) durch Behandlung der Verbindungen der Formel (V): 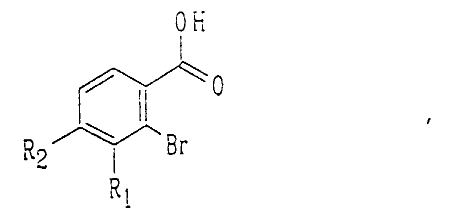 worin R₁ und R₂ die gleiche Bedeutung wie in Formel (I) besitzen, mit einem Mittel zur Aktivierung ihrer Säuregruppe hergestellt werden und das erhaltene Produkt in Gegenwart einer organischen oder anorganischen Base in einem organischen Lösungsmittel mit dem Amin HNR₃R₄ umgesetzt wird.

## Claims

1. Compound of formula (I): 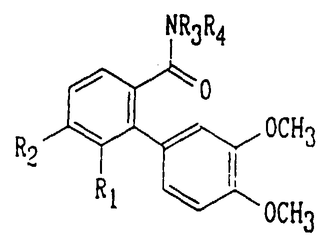 in which:
- R₁ and R₂, which are identical or different, are a hydrogen atom or an optionally halogenated alkyl radical containing 1 to 4 carbon atoms, or else R₁ and R₂ are each a chlorine atom, and
- R₃ and R₄, which are identical or different, are an alkyl containing 1 to 4 carbon atoms, or may form, together with the nitrogen atom which carries them, a morpholino ring.

2. Compound according to claim 1, characterised in that, in the formula I, one of R₁ and R₂ is a hydrogen atom and the other is the trifluoromethyl group.

3. Compound according to claim 1, characterised in that, in the formula I, R₁ and R₂ are each a chlorine atom.

4. Compound according to claim 1, characterised in that, in the formula I, R₃ and R₄, which are identical or different, are a methyl or an ethyl.

5. Compound according to claim 1, characterised in that, in the formula I, R₃ and R₄ form, together with the nitrogen atom which carries them, a morpholino ring.

6. Fungicidal composition for protecting plants, characterised in that it contains, as active material, a compound according to one of claims 1 to 5.

7. Process for protecting plants against fungal diseases, characterised in that a compound according to one of claims 1 to 5 is applied.

8. Process according to claim 7, characterised in that a curative treatment is carried out.

9. Process for the preparation of the compounds of formula (I), according to one of claims 1 to 5, characterised in that compounds of formula (II): 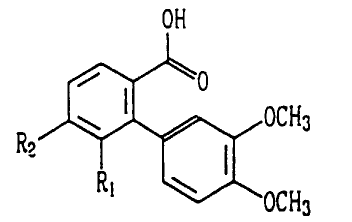 in which R₁ and R₂ have the same meaning as in the formula I,
are treated with an agent for activating their acidic functional group and in that the product obtained is reacted with an amine HNR₃R₄ in the presence of an organic or inorganic base in an organic solvent.

10. Process according to claim 9, characterised in that the agent for activating the acidic functional group is chosen from the group comprising thionyl chloride (SOCl₂), phosphoryl chloride (POCl₃), phosphorus trichloride or pentachloride (PCl₃, PCl₅), dicyclohexylcarbodiimide, carbonyldiimidazole, alkyl chloroformates and trifluoroacetic anhydride, and in that the reaction with the amine HNR₃R₄ is carried out in a solvent chosen from the group comprising chlorinated or aromatic solvents and ethers such as THF.

11. Process according to claim 9, characterised in that the compounds of formula (II) are prepared by saponification of the compounds of formula (III): 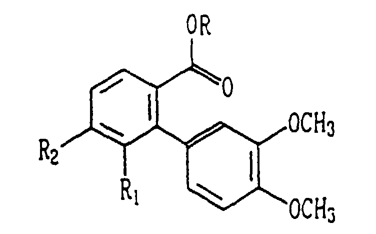 in which R₁ and R₂ have the same meaning as in the formula I and R is an alkyl containing 1 to 4 carbon atoms,
in an alcohol such as ethanol in the presence of water and of an alkali metal inorganic base, at a temperature between room temperature and the reflux temperature of the reaction mixture, the reaction mixture then being treated with an organic or inorganic acid such as hydrochloric acid.

12. Process according to claim 11, characterised in that the compounds of formula (III) are prepared by an aryl coupling reaction between a compound of formula (IV): 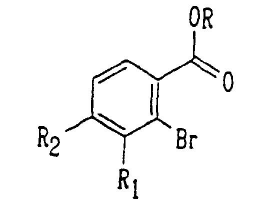 in which R, R₁ and R₂ have the same meaning as in the formula III and neither of the substituents R₁ and R₂ may be simultaneously a bromine or iodine atom,
and 3,4-dimethoxyphenylboronic acid in the presence of a catalyst.

13. Process for the preparation of the compounds of formula (I), characterised in that an aryl coupling reaction is carried out between a compound of formula (VII): 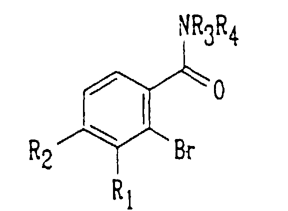 in which R₁ and R₂ have the same meaning as in the formula III and may not be simultaneously a bromine or iodine atom,
and 3,4-dimethoxyphenylboronic acid in the presence of a catalyst.

14. Process according to claim 13, characterised in that the compounds of formula (VII) are prepared by treating compounds of formula (V): 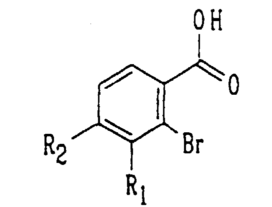 in which R₁ and R₂ have the same meaning as in the formula I,
with an agent for activating their acidic functional group and in that the product obtained is reacted with an amine HNR₃R₄ in the presence of an organic or inorganic base in an organic solvent.
